# EUROPÄISCHE PATENTSCHRIFT

(11) **EP 2 850 995 B1**
(45) Veröffentlichungstag und Bekanntmachung des Hinweises auf die Patenterteilung: **25.07.2018**
(21) Anmeldenummer: 14184551.1
(22) Anmeldetag: 12.09.2014
(51) Int. Cl.: A61B 1/00

(54) **Endoskop**
Endoscope
Endoscope

(30) Priorität: 20.09.2013 DE 102013110423
(43) Veröffentlichungstag der Anmeldung: 25.03.2015
(73) Patentinhaber: Karl Storz SE & Co. KG, 78532 Tuttlingen (DE)
(72) Erfinder: Vogel, Walter, 78532 Tuttlingen (DE)

(56) Entgegenhaltungen:
- EP-A1- 2 574 268
- WO-A2-02/01934
- US-A- 5 568 312
- US-A- 5 892 630
- US-A1- 2008 249 367

## Beschreibung

Die vorliegende Erfindung ist auf ein Endoskop und insbesondere auf eine Endoskop mit einstellbarer Blickrichtung und einem nicht-sphärischen Fensterbauteil bezogen.

Ein Endoskop weist am distalen Ende ein Fensterbauteil aus einem transparenten Material auf, durch das von einem zu beobachtenden Gegenstand ausgehendes Licht in das distale Ende des Endoskops eintreten kann. Licht, das durch das Fensterbauteil in das distale Ende des Endoskops eingetreten ist, kann mittels eines Objektivs abgebildet und mittels einer Kamera in ein elektrisches Signal gewandelt oder mittels eines geordneten Bündels von Lichtleitfasern oder mittels eines Relaislinsensystems zum proximalen Ende des Endoskops übertragen zu werden. Insbesondere bei Endoskopen für medizinische Anwendungen verschließt das Fensterbauteil das distale Ende des Endoskops hermetisch dicht, um das Eindringen von Schmutz, Wasserdampf und anderen störend oder zerstörend wirkenden Substanzen in das Endoskop zu vermeiden.

Bei Endoskopen mit feststehender Blickrichtung, d.h. mit unveränderlichem Winkel zwischen Blickrichtung und Längsachse des Schafts, ist das Fensterbauteil in der Regel eine Platte mit zwei ebenen und parallelen Oberflächen oder zumindest mit einer Rotationssymmetrie zur optischen Achse bzw. zur Blickrichtung. Bei einem Endoskop mit einstellbarer Blickrichtung, d.h. mit einem veränderbaren Winkel zwischen der Blickrichtung und der Längsachse des Schafts, kann das Fensterbauteil die Gestalt eines Ausschnitts eines Kreiszylindermantels aufweisen, wobei die Zylinderachse parallel zur Schwenkachse der Blickrichtung, orthogonal zur Blickrichtung und orthogonal zur Längsachse des Schafts ist.

Ein Zylinder im hier gemeinten geometrischen Sinne ist ein Körper, der durch zwei ebene und parallele Flächen, die auch als Grund- und Deckfläche bezeichnet werden, und durch eine Mantel- bzw. Zylinderfläche begrenzt wird. Die ebenen und parallelen Grund- und Deckfläche spielen im Folgenden keine Rolle und sind insbesondere durch Schleifen oder auf andere Weise entfernt oder verändert. Die Mantel- bzw. Zylinderfläche wird von parallelen Geraden, die einander entsprechende Punkte an den Rändern der ebenen Flächen verbinden, gebildet. Ein Zylinder entsteht also durch Verschiebung einer ebenen Fläche oder Kurve entlang einer Geraden, die nicht in der Ebene liegt. Grund- und Deckfläche können kreisförmig sein oder eine beliebige andere Gestalt aufweisen. Wenn die Grundfläche und die Deckfläche kreisförmig sind, ist der Zylinder ein Kreiszylinder. Wenn die die Mantel- bzw. Zylinderfläche bildendenden Geraden senkrecht zu den ebenen Flächen sind, wird der Zylinder als gerader Zylinder bezeichnet.

Mit einer Zylinderachse eines Zylinders ist hier jede Gerade gemeint, die parallel zu den Geraden, die die Mantel- bzw. Zylinderfläche bilden, ist. Die Zylinderachse gibt die Richtung an, in der der Zylinder innerhalb gewisser Grenzen translationsinvariant ist. Die Zylinderachse eines geraden Kreiszylinders ist die Gerade, auf der die Mittelpunkte von Grund- und Deckfläche liegen. Der gerade Kreiszylinder ist rotationssymmetrisch zu dieser Symmetrieachse.

Mit voranschreitender Miniaturisierung auch von Endoskopen mit einstellbarer Blickrichtung wächst das Verhältnis zwischen der Wandstärke und dem Krümmungsradius des Fensterbauteils. Damit wachsen die von dem Fensterbauteil erzeugten Aberrationen, insbesondere der Astigmatismus, auch auf der optischen Achse. Um diese Abbildungsfehler zu vermeiden, wird ein sphärisches Fensterbauteil verwendet. Seitlich des sphärschen Fensterbauteils sind oft überstehende Ränder vorgesehen, die die Fertigung, insbesondere das Fügen des sphärischen Fensterbauteils mit dem distalen Ende des Mantels des Schafts, vereinfachen können. Diese überstehenden Ränder können ferner ein Überstehen des sphärischen Fensterbauteils verhindern und damit das Risiko einer Beschädigung oder Zerstörung mindern. In den resultierenden konkaven Abschnitten können sich jedoch Verunreinigungen absetzen, die die Sicht beeinträchtigen und eine Reinigung des Endoskops erschweren können.

Die US 5 892 630 A zeigt ein Endoskop mit einem transparenten ebenen Fensterbauteil, das am distalen Ende des Endoskops vorliegt. Darüber hinauf offenbart dieses Dokument zur Korrektur der Aberration des Fensterbauteils eine optische Korrektureinrichtung in Form eines Prismas.

In der EP 2 574 268 A1 ist ein Endoskop gezeigt, das ebenso ein transparentes Fensterbauteil am distalen Endoskopende aufweist. Dieses Fensterbauteil hat die Form eines Ausschnitts eines Kreiszylindermantels.

Eine Aufgabe der vorliegenden Erfindung besteht darin, ein verbessertes Endoskop, insbesondere ein verbessertes Endoskop mit einer einstellbaren Blickrichtung zu schaffen. Diese Aufgabe wird durch die Gegenstände der unabhängigen Ansprüche gelöst. Weiterbildungen sind in den abhängigen Ansprüchen angegeben.

Ein Endoskop umfasst ein Fensterbauteil aus einem transparenten Material am distalen Ende des Endoskops, wobei das Fensterbauteil nicht rotationssymmetrisch zur Blickrichtung ist, und eine optische Korrektureinrichtung zur Korrektur der Aberration des nicht-sphärischen Fensterbauteils.

Das Endoskop ist für medizinische, technische oder andere nicht-medizinische Anwendungen vorgesehen und ausgebildet. Das Fensterbauteil weist ein Material auf, das für Licht im für das menschliche Auge sichtbaren Wellenlängenbereich und/oder für Licht in einem anderen Wellenlängenbereich, in dem mittels des Endoskops ein Gegenstand beobachtet werden soll, eine hohe Transmission aufweist. Das Fensterbauteil ist insbesondere nicht-sphärisch. Ein Fensterbauteil ist nicht-sphärisch, wenn zumindest entweder seine innere (proximale) oder seine äußere (distale) Oberfläche nicht sphärisch bzw. nicht kugelförmig ist. Eine ebene Oberfläche ist eine sphärische Oberfläche mit Radius unendlich.

Die optische Korrektureinrichtung ist insbesondere zur Korrektur eines axialen Astigmatismus des Fensterbauteils vorgesehen und ausgebildet. Die optische Korrektureinrichtung kann eine oder mehrere Linsen oder andere optische Elemente umfassen. Die optische Korrektureinrichtung kann als mechanisch und/oder optisch eigenständige Einheit, die ausschließlich der Korrektur der Aberration des nicht-sphärischen Fensterbauteils dient, ausgebildet sein. Alternativ kann die optische Korrektureinrichtung teilweise oder vollständig in eine andere optische Einrichtung, beispielsweise in ein Objektiv, eine Stablinse oder ein Okular, integriert sein.

Die optische Korrektureinrichtung zur Korrektur der Aberration ermöglicht insbesondere die Verwendung eines nicht-sphärischen Fensterbauteils, das gegenüber einem sphärischen Fensterbauteil unter anderem Vorteile hinsichtlich der mechanischen Robustheit, des Herstellungsaufwands, der Herstellungskosten und des Montageaufwands aufweisen kann. Gleichzeitig ermöglicht die Verwendung der optischen Korrektureinrichtung optische Eigenschaften und insbesondere eine Abbildungsqualität, die denen eines sphärischen Fensterbauteils zumindest nahekommen.

Bei einem Endoskop, wie es hier beschrieben ist, ist insbesondere die Blickrichtung des Endoskops einstellbar.

Mit einer Einstellbarkeit der Blickrichtung ist insbesondere eine Veränderbarkeit des Winkels zwischen der Blickrichtung und der Längsachse des Schafts des Endoskops an dessen distalem Ende gemeint. Insbesondere bei einem Endoskop mit einstellbarer Blickrichtung kann es wünschenswert und vorteilhaft sein, ein Fensterbauteil zu verwenden, das nicht rotationssymmetrisch zur Blickrichtung und insbesondere nicht-sphärisch ist.

Bei einem Endoskop, wie es hier beschrieben ist, weist das Fensterbauteil insbesondere die Gestalt eines Ausschnitts eines Kreiszylindermantels auf.

Das Fensterbauteil weist insbesondere im Wesentlichen die Gestalt eines rechteckigen Ausschnitts eines Kreiszylindermantels auf, wobei zwei einander gegenüberliegende Randabschnitte des Fensterbauteils jeweils gerade und parallel zueinander und zwei weitere einander gegenüberliegende Randabschnitte des Fensterbauteils jeweils kreisbogenförmig und zueinander parallel sind. Die innere und die äußere Oberfläche des Fensterbauteils weisen jeweils die Gestalt eines Ausschnitts einer Oberfläche eines Kreiszylinders auf, wobei beide Kreiszylinder insbesondere unterschiedliche Radien aufweisen, und wobei die Symmetrieachsen der beiden Kreiszylinder insbesondere zusammenfallen.
Ein Fensterbauteil in Gestalt eines Ausschnitts eines Kreiszylindermantels kann besonders kostengünstig herstellbar sein. Insbesondere wird das Fensterbauteil aus einem zylindrischen Rohr geschnitten. Auch eine reflexmindernde Beschichtung und/oder eine andere Vergütung der Oberflächen des Fensterbauteils kann bei einer Ausgestaltung als Ausschnitt eines Kreiszylindermantels in besonders hoher Qualität und/oder mit vergleichsweise geringem Aufwand und deshalb kostengünstig herstellbar sein. Ferner kann ein Fensterbauteil in Gestalt eines Ausschnitts eines Kreiszylindermantels besonders einfach und/oder präzise lötbar oder anderweitig mit dem Schaft des Endoskops fügbar sein. Ferner kann ein Fensterbauteil in Gestalt eines Ausschnitts eines Kreiszylindermantels zusammen mit umgebenden Bereichen der äußeren Oberfläche des Schafts des Endoskops eine besonders glatte Fläche ohne Spalte oder andere konkave Bereiche bilden.

Bei einem Endoskop, wie es hier beschrieben ist, sind das nicht-sphärische Fensterbauteil und die optische Korrektureinrichtung zusammen insbesondere zu einem sphärischen transparenten Bauteil optisch äquivalent. Anders ausgedrückt wird ein fiktives sphärisches transparentes Bauteil in zwei zusammengenommen optisch äquivalente oder im Wesentlichen äquivalente Bauteile gesplittet bzw. aufgetrennt, nämlich in das Fensterbauteil einerseits und die optische Korrektureinrichtung andererseits. Dabei sind insbesondere beide Bauteile jeweils zylindrisch oder im Wesentlichen zylindrisch. Die Zylinderachsen beider Bauteile sind insbesondere parallel zueinander. Alternativ können die Zylinderachsen beider Bauteile orthogonal zueinander sein.

Bei einem Endoskop, wie es hier beschrieben ist, umfasst die Korrektureinrichtung insbesondere eine Zylinderlinse.

Insbesondere sind das Fensterbauteil als Ausschnitt eines Kreiszylindermantels und die Zylinderlinse als sammelnde Zylinderlinse ausgebildet, wobei die Zylinderachse der Zylinderlinse parallel zur Zylinderachse des Fensterbauteils ist.

Die Zylinderlinse kann sowohl konvex als auch konkav ausgebildet sein. Wenn das nicht-sphärische Fensterbauteil die Gestalt eines Ausschnitts eines Kreiszylindermantels aufweist, ist die Zylinderlinse insbesondere konvex, wenn die Zylinderachse der Zylinderlinse parallel zur Zylinderachse des Kreiszylindermantels ist und konkav, wenn die Zylinderachse der Zylinderlinse orthogonal zur optischen Achse und orthogonal zur Zylinderachse des Kreiszylindermantels ist.

Die Zylinderachse einer Zylinderlinse gibt die Richtung an, zu der sowohl die Lichteintrittsfläche als auch die Lichtaustrittsfläche jeweils parallel sind. Anders ausgedrückt sind sowohl die Lichteintrittsfläche als auch die Lichtaustrittsfläche jeweils durch eine Schar von Geradenabschnitten gebildet, die parallel zur Zylinderachse sind. Deck- und Bodenfläche eines idealen Zylinders im geometrischen Sinne spielen an einer Zylinderlinse keine Rolle. In der Regel sind Deck- und Bodenfläche entfernt und die Zylinderlinse in einer Ebene senkrecht zur Ausbreitungsrichtung des Lichts und parallel zur Zylinderachse der Zylinderlinse an einen insbesondere kreisförmigen Querschnitt benachbarter Linsen angepasst.

Eine Zylinderlinse ist konvex, wenn sie in der Mitte bzw. an der optischen Achse dicker ist als am Rand. Insbesondere sind die Lichteintrittsfläche und die Lichtaustrittsfläche einer konvexen Zylinderlinse jeweils konvex. Alternativ ist eine der beiden Lichtein- und -austrittsflächen konvex, die andere konkav, wobei die Krümmung der konvexen größer ist als die der konkaven.

Eine Zylinderlinse ist konkav, wenn sie in der Mitte bzw. an der optischen Achse dünner ist als am Rand. Insbesondere sind die Lichteintrittsfläche und die Lichtaustrittsfläche einer konkaven Zylinderlinse jeweils konkav. Alternativ ist eine der beiden Lichtein- und -austrittsflächen konvex, die andere konkav, wobei die Krümmung der konkaven größer ist als die der konvexen.

Bei einem Endoskop, wie es hier beschrieben ist, ist die Korrektureinrichtung insbesondere nahe dem Fensterbauteil angeordnet.

Insbesondere ist die Korrektureinrichtung distal eines Stablinsensystems oder eines anderen Relaislinsensystems angeordnet. Die distale Anordnung der Korrektureinrichtung ermöglicht eine Übertragung eines bereits hinsichtlich der Aberration des nicht-sphärischen Fensterbauteils korrigierten Bilds mittels des Stablinsen- oder Relaislinsensystems. Ein Endoskop, wie es hier beschrieben ist, umfasst insbesondere ferner eine schwenkbare optische Einrichtung zum Einstellen der Blickrichtung des Endoskops, wobei die Korrektureinrichtung proximal der schwenkbaren optischen Einrichtung angeordnet ist.

Die schwenkbare optische Einrichtung umfasst insbesondere eine oder mehrere reflektierende Flächen, von denen mindestens eine um eine Achse orthogonal zur Längsachse des Schafts des Endoskops an dessen distalem Ende und orthogonal zu allen einstellbaren Blickrichtungen schwenkbar ist. Die schwenkbare optische Einrichtung umfasst insbesondere ein schwenkbares Dove-Prisma oder zwei insbesondere jeweils um 90 Grad umlenkende Umlenkprismen, von denen eines schwenkbar ist. Die Korrektureinrichtung ist insbesondere unmittelbar proximal des schwenkbaren Dove-Prismas oder unmittelbar proximal des feststehenden von zwei Umlenkprismen angeordnet.

Ein Endoskop, wie es hier beschrieben ist, umfasst insbesondere ferner ein Objektiv am distalen Ende des Endoskops, wobei die Korrektureinrichtung in das Objektiv integriert oder unmittelbar distal oder proximal des Objektivs angeordnet ist.

Insbesondere ist die Korrektureinrichtung zwischen dem Objektiv und dem distalen Ende eines Stablinsensystems oder eines anderen Relaislinsensystems angeordnet. Dies kann einen herkömmlich kleinen Abstand zwischen dem Objektiv einerseits und dem nicht-sphärischen Fensterbauteil und einer schwenkbaren optischen Einrichtung proximal desselben andererseits ermöglichen.

Ein Endoskop, wie es hier beschrieben ist, umfasst insbesondere ferner ein Relaislinsensystem zur Übertragung eines Bilds vom distalen Ende zum proximalen Ende des Endoskops, wobei die Korrektureinrichtung in das Relaislinsensystem integriert oder zwischen zwei Teilen des Relaislinsensystems angeordnet ist.

Das Relaislinsensystem ist insbesondere ein Stablinsensystem aus einer Mehrzahl von Stablinsen. Die Korrektureinrichtung ist insbesondere zwischen zwei Stablinsen angeordnet. Alternativ ist eine Stablinse gleichzeitig als Korrektureinrichtung ausgebildet. Dazu weist die Stablinse insbesondere einen Astigmatismus oder eine andere Abweichung von einer perfekten Rotationssymmetrie bezüglich der optischen Achse auf, der zur Korrektur der Aberration des nicht-sphärischen Fensterbauteils geeignet ist.

Bei einem Endoskop, wie es hier beschrieben ist, ist die Korrektureinrichtung insbesondere an oder nahe dem proximalen Ende des Endoskops angeordnet.

Ein Endoskop, wie es hier beschrieben ist, umfasst insbesondere ferner ein Okular am proximalen Ende des Endoskops, wobei die Korrektureinrichtung in das Okular integriert oder unmittelbar distal oder unmittelbar proximal des Okulars angeordnet ist.

Eine Anordnung der Korrektureinrichtung am oder nahe dem proximalen Ende des Endoskops und insbesondere unmittelbar distal oder unmittelbar proximal des Okulars oder eine Ausbildung der Korrektureinheit als Teil des Okulars ermöglicht bei einer Fertigung des Endoskops von distal nach proximal eine Korrektur weiterer Aberrationen mittels der Korrektureinrichtung. Zu diesen weiteren, durch die Korrektureinrichtung korrigierbaren Aberrationen können Fehler zählen, die durch eine imperfekte bzw. mangelhafte (beispielsweise verkippte) Montage des Fensterbauteils, einer schwenkbaren optischen Einrichtung, eines Objektivs, eines Relaislinsensystems oder von Teilen eines Relaislinsensystems hervorgerufen werden. Eine Anordnung der Korrektureinrichtung an oder nahe dem proximalen Ende des Endoskops und eine entsprechend späte Montage der Korrektureinrichtung kann die optische Qualität des gefertigten Endoskops erhöhen und den Anteil der Fehlproduktion oder des Ausschusses und damit die gesamten Herstellungskosten vermindern.

Bei einem Endoskop, wie es hier beschrieben ist, weist die Korrektureinrichtung insbesondere eine einstellbare Korrekturwirkung auf, um eine Anpassbarkeit der Korrekturwirkung an den Brechungsindex eines Mediums, in dem das distale Ende des Endoskops angeordnet ist, zu ermöglichen.

Endoskope werden, insbesondere bei medizinischen Anwendungen, häufig abwechselnd in Luft, Kohlendioxid oder einem anderen Gas und in Wasser, wässrigen Lösungen oder anderen transparenten Flüssigkeiten verwendet. Die Abhängigkeit der Brechungswirkung der äußeren Oberfläche des Fensterbauteils vom Brechungsindex des umgebenden Mediums ist in vielen Fällen mittels der zur Fokussierung bzw. Scharfstellung vorgesehenen Einrichtung ohne Weiteres kompensierbar. Der Brechungsindex des umgebenden Mediums hat jedoch auch Einfluss auf die Aberration, insbesondere den Astigmatismus, eines nicht-sphärischen Fensterbauteils. In einem Medium mit Brechungsindex 1 (Luft oder andere Gase) und in einem Medium mit einem Brechungsindex > 1 (Wasser oder eine andere Flüssigkeit) sind deshalb unterschiedliche Aberrationen zu korrigieren. Eine einstellbare Korrekturwirkung der Korrektureinrichtung kann eine Verwendung des Endoskops mit perfekt oder im Wesentlichen perfekt korrigierter Aberration des nicht-sphärischen Fensterbauteils in Medien mit unterschiedlichen Brechungsindizes ermöglichen.

Bei einem Endoskop, wie es hier beschrieben ist, ist die Korrekturwirkung der Korrektureinrichtung insbesondere zwischen einer ersten vorbestimmten Korrekturwirkung für die Verwendung des distalen Endes des Endoskops in Luft oder einem anderen Gas und einer zweiten vorbestimmten Korrekturwirkung für die Verwendung des distalen Endes des Endoskops in Wasser oder einer Flüssigkeit mit ähnlichem Brechungsindex umschaltbar.

Insbesondere weist das nicht-sphärische Fensterbauteil in Luft oder einem anderen Gas einerseits und in Wasser oder einer Flüssigkeit mit ähnlichem Brechungsindex andererseits zwei unterschiedliche Werte des Astigmatismus auf. Die Korrektureinrichtung ist umschaltbar zwischen zwei zur Korrektur je eines der beiden Astigmatismus-Werte vorgesehenen Korrekturwirkungen. Insbesondere umfasst die Korrektureinrichtung mindestens ein optisches Element, das zwischen zwei vorbestimmten Positionen (parallel zur optischen Achse) verschiebbar oder (um die optische Achse) rotierbar ist. Die beiden vorbestimmten Positionen sind insbesondere durch mechanische Anschläge oder Rastpositionen definiert.

Kurzbeschreibung der Figuren

Nachfolgend werden Ausführungsformen anhand der beigefügten Figuren näher erläutert. Es zeigen:
Figur 1 eine schematische Darstellung eines Endoskops;
Figur 2 eine schematische Darstellung eines Teils des Endoskops aus Figur 1;
Figur 3 eine schematische Darstellung eines Teils eines weiteren Endoskops;
Figur 4 eine schematische Darstellung eines Teils eines weiteren Endoskops;
Figur 5 eine schematische Darstellung eines Teils eines weiteren Endoskops;
Figur 6 eine schematische Darstellung eines Teils eines weiteren Endoskops.

### Beschreibung der Ausführungsformen

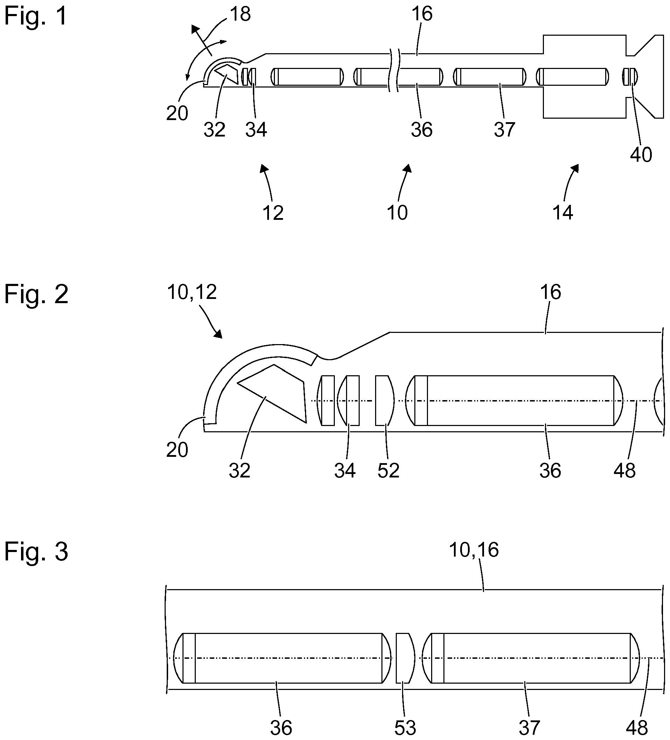

Figur 1 zeigt eine schematische Darstellung eines Endoskops 10 mit einem distalen Ende 12 und einem proximalen Ende 14. Ein gerader und starrer Schaft 16 erstreckt sich vom distalen Ende 12 bis zum proximalen Ende 14 des Endoskops 10. Die Blickrichtung 18 des Endoskops 10 ist innerhalb eines in Figur 1 durch einen gebogenen Doppelpfeil angedeuteten Winkelbereichs einstellbar.

Das Endoskop 10 und seine bisher beschriebenen Merkmale sind in Figur 1 im Wesentlichen durch Konturen angedeutet. Die nachfolgend beschriebenen optischen Einrichtungen sind hingegen in einer Seitenansicht angedeutet. Dabei sind nicht nur die äußeren Konturen der optischen Einrichtungen, wie sie bei einem Schnitt entlang einer Ebene, die die optische Achse enthält, sichtbar werden, sondern auch Kanten, die sich insbesondere kreisbogenförmig aus der Zeichenebene herauswölben, in Projektion auf die Zeichenebene dargestellt. Insbesondere sind kreisbogenförmige Kanten zwischen gewölbten Lichteintrittsflächen oder Lichtaustrittsflächen einerseits und zylindrischen Mantelflächen andererseits als gerade Linien orthogonal zur Längsachse des Endoskops 10 erkennbar, da diese kreisbogenförmigen Kanten in Ebenen orthogonal zur optischen Achse und damit auch orthogonal zur Längsachse des Endoskops 10 und zur Zeichenebene liegen. Ferner sind als gerade Linien die ebenfalls kreisbogenförmigen Ränder von Grenzflächen zwischen miteinander verkitteten Elementen erkennbar.

Das Endoskop 10 umfasst am distalen Ende 12 ein Fensterbauteil 20 aus einem Material, das insbesondere für Licht innerhalb des für das menschliche Auge sichtbaren Wellenlängenbereichs transparent ist bzw. eine möglichst hohe Transmission aufweist. Das Fensterbauteil 20 weist die Gestalt eines Ausschnitts eines Kreiszylindermantels auf, wobei die Zylinderachse des Kreiszylindermantels orthogonal zur optischen Achse weiterer optischer Einrichtung des Endoskops 10, orthogonal zur Längsachse des Schafts 16, orthogonal zur Blickrichtung 18 des Endoskops und orthogonal zur Zeichenebene ist. Die äußere Oberfläche des Fensterbauteils 20 ist Lichteintrittsfläche, die innere Oberfläche des Fensterbauteils 20 ist Lichtaustrittsfläche für Licht, das von einem näherungsweise in der Blickrichtung 18 des Endoskops 10 liegenden, zu beobachtenden Gegenstand ausgeht und durch das Fensterbauteil 20 hindurch in das Endoskop 10 eintritt. Die äußere Eintrittsfläche und die innere Lichtaustrittsfläche des Fensterbauteils 20 weisen jeweils die Gestalt eines Ausschnitts einer Oberfläche eines Kreiszylinders auf, wobei die Zylinderachsen beider Kreiszylinder zusammenfallen, so dass das Fensterbauteil 20 eine konstante Wandstärke aufweist. Das Fensterbauteil 20 ist mit dem Schaft 16 des Endoskops 10 so durch Löten oder auf andere Weise gefügt, dass es eine Öffnung am distalen Ende 12 des Endoskops 10 hermetisch dicht verschließt.

Unmittelbar proximal und lichtstromabwärts des Fensterbauteils 20 ist ein Schwenkprisma 32 angeordnet. Das Schwenkprisma 32 weist insbesondere die Gestalt eines Dove-Prismas auf und ist um eine nicht dargestellte Schwenkachse orthogonal zur Zeichenebene der Figur 1 schwenkbar, um die Blickrichtung 18 einzustellen.

Lichtstromabwärts des Schwenkprismas 32 ist ein Objektiv 34 aus einer oder mehreren Linsen oder eine andere Abbildungseinrichtung zur Erzeugung eines Zwischenbilds eines mittels des Endoskops 10 beobachteten Gegenstands angeordnet. Proximal und lichtstromabwärts des Objektivs 34 ist ein Stablinsensystem aus mehreren Stablinsen 36, 37 oder ein anderes Relaislinsensystem zur Übertragung des vom Objektiv 34 erzeugten Zwischenbilds zum distalen Ende 14 des Endoskops 10 im Schaft 16 angeordnet. Proximal und lichtstromabwärts des Stablinsensystems 36, 37 ist ein Okular 40 zum Erzeugen eines virtuellen Bilds, das vom menschlichen Auge erfassbar ist, angeordnet. Das Okular 40 kann eine oder mehrere Linsen umfassen und gleichzeitig das Endoskop 10 am proximalen Ende 14 hermetisch dicht verschließen.
Das Fensterbauteil 20 am distalen Ende 12 des Endoskops 10 erzeugt aufgrund seiner nicht-sphärischen, sondern kreiszylindermantelförmigen Gestalt Abbildungsfehler bzw. Aberrationen, die umso größer sind, je größer das Verhältnis zwischen der Wanddicke und dem Krümmungsradius des Fensterbauteils 20 ist und je größer der Durchmesser eines mittels des Schwenkprismas, des Objektivs 34, des Stablinsensystems 36, 37 und des Okulars 40 erfassten Lichtbündels ist. Insbesondere weist das Fensterbauteil 20 einen axialen Astigmatismus auf. Zur Korrektur des Astigmatismus können das Objektiv 34, eine oder mehrere der Stablinsen 36, 37 oder das Okular 40 als Korrektureinrichtung ausgebildet oder eine oder mehrere Korrektureinrichtungen, die in Figur 1 nicht dargestellt sind, vorgesehen sein.

Figur 2 zeigt eine schematische Darstellung eines distalen Endes 12 eines weiteren Endoskops, das in einigen Merkmalen und Eigenschaften dem oben anhand der Figur 1 dargestellten Endoskop ähnelt. Die Zeichenebene und die Art der Darstellung in Figur 2 entsprechen denjenigen der Figur 1. Nachfolgend sind lediglich Merkmale und Eigenschaften beschrieben, in denen sich das Endoskop von dem oben anhand der Figur 1 dargestellten Endoskop unterscheidet.

Am distalen Ende 12 des Endoskops ist eine Korrektureinrichtung in Gestalt einer Korrekturlinse 52 unmittelbar proximal des Objektivs 34 und unmittelbar distal der äußerst distalen Stablinse 36 und somit zwischen dem Objektiv 34 und der äußerst distalen Stablinse 36 angeordnet. Die Korrekturlinse 52 ist als Zylinderlinse ausgebildet, deren Zylinderachse orthogonal zur optischen Achse 48 des Objektivs 34 und der Stablinse 36, parallel zur Zylinderachse des Fensterbauteils 20 und orthogonal zur Zeichenebene der Figur 2 ist. Die Korrekturlinse 52 ist insbesondere als sammelnde Zylinderlinse, die in der dargestellten Projektion in der Mitte dicker ist als außen, ausgebildet. In einer nicht dargestellten Projektion parallel zur optischen Achse 48 des Objektivs 34 und der Stablinsen 36 weist die Korrekturlinse 52 insbesondere die gleiche kreisförmige Kontur wie das Objektiv 34 und/oder wie die Stablinse 36 auf.

Die Korrekturlinse 52 ist zur Korrektur der Aberration des nicht-sphärischen Fensterbauteils 20, insbesondere zur Korrektur von dessen Astigmatismus vorgesehen und ausgebildet. Die Anordnung der Korrekturlinse 52 am distalen Ende 12 des Endoskops kann beispielsweise dahin gehend vorteilhaft sein, dass von den Stablinsen 36 bereits ein korrigiertes Bild übertragen wird. Ferner ermöglicht die Anordnung der Korrekturlinse 52 am distalen Ende 12 eine Verwendung eines geordneten Bündels von Lichtleitfasern anstelle der Stablinsen 36 und damit auch die Verwendung eines flexiblen Schafts 16.

Abweichend von der Darstellung in Figur 2 kann die Korrekturlinse 52 distal des Objektivs 34 bzw. zwischen dem Schwenkprisma 32 und dem Objektiv 34 oder zwischen Linsen des Objektivs 34 angeordnet sein. Ferner können abweichend von der Darstellung in Figur 2 anstelle einer Korrekturlinse 52 eine oder mehrere Linsen des Objektivs 34 so ausgebildet sein, dass sie die Aberration, insbesondere den Astigmatismus des Fensterbauteils 20 korrigieren.

Figur 3 zeigt eine schematische Darstellung eines Teils eines Schafts 16 eines weiteren Endoskops, das in einigen Merkmalen und Eigenschaften den oben anhand der Figuren 1 und 2 dargestellten Endoskopen ähnelt. Die Zeichenebene und die Art der Darstellung entsprechen denen der Figuren 1 und 2. Nachfolgend sind lediglich Merkmale und Eigenschaften beschrieben, in denen sich das Endoskop 10 von den oben anhand der Figuren 1 und 2 dargestellten Endoskopen unterscheidet.

Das Endoskop 10 unterscheidet sich von den oben anhand der Figuren 1 und 2 dargestellten Endoskopen insbesondere dadurch, dass eine Korrekturlinse 53 zwischen zwei Stablinsen 36, 37 eines Stablinsensystems angeordnet ist. Die Korrekturlinse 53 kann ähnliche Merkmale und Eigenschaften aufweisen wie die Korrekturlinse 52 des oben anhand der Figur 2 dargestellten Endoskops 10.

Abweichend von der Darstellung in Figur 3 kann anstelle der Korrekturlinse 53 zwischen den Stablinsen 36, 37 eine die Aberration des Fensterbauteils 20 korrigierende Wirkung von einer oder mehreren Stablinsen 36, 37 vorgesehen sein. Insbesondere können eine oder mehrere Stablinsen 36, 37 von einer idealen Rotationssymmetrie bezüglich der optischen Achse 48 abweichen und einen Astigmatismus aufweisen, der den Astigmatismus des nicht-sphärischen Fensterbauteils 20 (vgl. Figuren 1, 2) korrigiert.

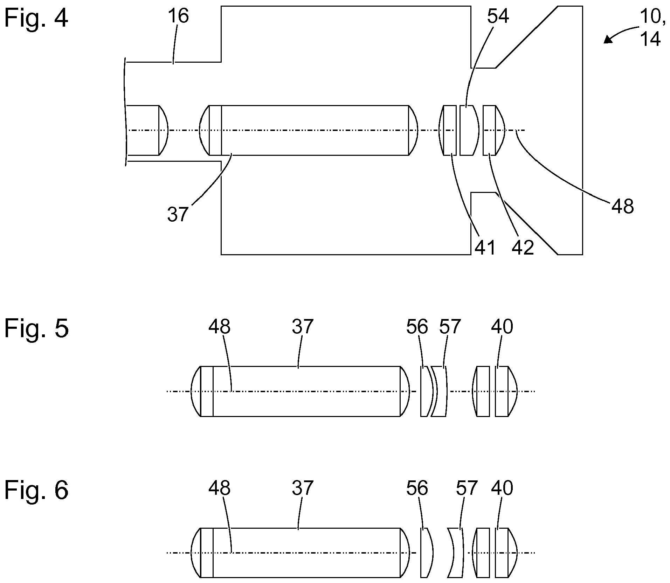

Figur 4 zeigt eine schematische Darstellung eines proximalen Endes 14 eines weiteren Endoskops 10, das in einigen Merkmalen und Eigenschaften den oben anhand der Figuren 1 bis 3 dargestellten Endoskopen ähnelt. Die Zeichenebene und die Art der Darstellung entsprechen denen der Figuren 1 bis 3. Nachfolgend sind lediglich Merkmale und Eigenschaften beschrieben, in denen sich das Endoskop 10 von den oben anhand der Figuren 1 bis 3 dargestellten unterscheidet.

Das Endoskop 10 weist am proximalen Ende 14 eine Korrekturlinse 54 zur Korrektur der Aberration eines in Figur 4 nicht dargestellten nicht-sphärischen Fensterbauteils 20 am distalen Ende 12 (vgl. Figur 1) auf. Die Korrekturlinse 54 ist insbesondere zwischen Linsen 41, 42 eines Okulars angeordnet. Die Korrekturlinse 54 weist insbesondere Merkmale und Eigenschaften ähnlich denen der oben anhand der Figuren 2 und 3 dargestellten Korrekturlinsen 52, 53 auf.

Abweichend von der Darstellung in Figur 4 kann die Korrekturlinse 54 am proximalen Ende 14 entweder proximal aller Linsen 41, 42 des Okulars oder distal aller Linsen 41, 42 des Okulars und damit zwischen der äußerst proximalen Stablinse 37 und dem Okular 41, 42 angeordnet sein. Ferner kann alternativ und abweichend von der Darstellung in Figur 4 anstelle einer Korrekturlinse 54 eine die Aberration des nicht-sphärischen Fensterbauteils korrigierende Wirkung von einer oder mehreren Linsen 41, 42 des Okulars vorgesehen sein. Dazu weist insbesondere mindestens eine der Linsen 41, 42 abweichend von der Darstellung in Figur 4 eine von einer idealen Rotationssymmetrie zur optischen Achse 48 abweichende Gestalt auf.

Die Anordnung einer Korrekturlinse 54 am proximalen Ende 14 eines Endoskops 10 kann insbesondere dann vorteilhaft sein, wenn das Endoskop 10 vom distalen Ende 12 (vgl. Figur 1) zum proximalen Ende 14 fortschreitend zusammengebaut wird. In diesem Fall können mittels der Korrekturlinse 54 oder einer entsprechenden Korrekturwirkung von einer oder mehreren Linsen 41, 42 des Okulars neben einer Aberration eines nicht-sphärischen Fensterbauteils 20 auch weitere Abbildungsfehler korrigiert werden, die beispielsweise von einer unpräzisen und nicht mehr korrigierbaren Anordnung oder Ausrichtung des Fensterbauteils 20, des Prismas 32, des Objektivs 34 oder einer Stablinse 36, 37 (vgl. Figur 1) oder von deren nicht-idealen Eigenschaften herrühren.

Ferner kann eine proximale Anordnung der Korrekturlinse 54 eine Rotation derselben beim Schwenken der Blickrichtung 18 (vgl. Figur 1) ermöglichen. Dies kann insbesondere vorteilhaft sein, wenn anstelle eines schwenkbaren Dove Prismas 32, wie es in den Figuren 1 und 2 gezeigt ist, eine Anordnung aus zwei Umlenkprismen oder Umlenkspiegeln, von denen einer zum Einstellen der Blickrichtung 18 schwenkbar ist, vorgesehen ist. Bei dieser einem Periskop ähnlichen Anordnung geht ein Schwenken der Blickrichtung 18 mit einer Rotation des Bilds um die optische Achse einher. Eine Korrektur einer durch das Fensterbauteil 20 erzeugten Aberration erfordert eine der Rotation des Bilds entsprechende Rotation der Korrekturlinse 54. Diese Rotation der Korrekturlinse 54 kann im Fall einer Anordnung der Korrekturlinse 54 nahe dem proximalen Ende 14 des Endoskops 10 besonders einfach realisierbar sein.

Figur 5 zeigt eine schematische Darstellung optischer Einrichtungen eines weiteren Endoskops, das in einigen Merkmalen und Eigenschaften den oben anhand der Figuren 1 bis 4 dargestellten Endoskopen ähnelt. Die Zeichenebene und die Art der Darstellung in Figur 5 entsprechen oder ähneln denen der Figuren 1 bis 4, wobei abweichend von den Darstellungen in den Figuren 1 bis 4 lediglich optische Einrichtungen, nicht jedoch Konturen des Endoskops selbst dargestellt sind. Nachfolgend sind lediglich Merkmale und Eigenschaften beschrieben, in denen sich das Endoskop, insbesondere seine optischen Einrichtungen, von dem oben anhand der Figur 4 dargestellten Endoskop unterscheiden.

Anstelle einer einzigen Korrekturlinse 54 (vgl. Figur 4) sind zwei Korrekturlinsen 56, 57 vorgesehen, von denen zumindest eine parallel zur optischen Achse 48 der Stablinsen 37 und des Okulars 40 verschiebbar angeordnet ist. Die erste Korrekturlinse 56 und die zweite Korrekturlinse 57 sind ausgebildet, um zusammen eine Korrekturwirkung aufzuweisen, die von ihrem gegenseitigen Abstand abhängt. Die Korrekturlinsen 56, 57 sind insbesondere jeweils zylinderförmig, wobei die Zylinderachsen orthogonal zur optischen Achse 48 der Stablinse 37 und des Okulars 40 und orthogonal zur Zeichenebene der Figur 5 sind. Insbesondere ist eine der beiden Korrekturlinsen 56, 57 in der Zeichenebene streuend und die andere sammelnd.

Figur 6 zeigt eine weitere schematische Darstellung der oben anhand der Figur 5 dargestellten optischen Einrichtungen 37, 40, 56, 57 eines Endoskops. Die Darstellung in Figur 6 unterscheidet sich von der Darstellung in Figur 5 insbesondere dadurch, dass eine der beiden Korrekturlinsen 56, 57 relativ zur anderen Korrekturlinse parallel zur optischen Achse 48 der Stablinse 37 und des Okulars 40 verschoben ist. Dadurch weisen die Korrekturlinsen 56, 57 zusammen in der in Figur 5 dargestellten Konfiguration einerseits und in der in Figur 6 dargestellten Konfiguration andererseits unterschiedliche Korrekturwirkungen auf. Diese unterschiedlichen Korrekturwirkungen können eine Korrektur unterschiedlicher Aberrationen, die aus einer Anordnung des distalen Endes 12 des Endoskops 10 (vgl. Figur 1) in Medien mit unterschiedlichen Brechungsindizes resultieren, ermöglichen.

Die parallel zur optischen Achse 48 verschiebbare Korrekturlinse 57 ist insbesondere mit einem in den Figuren 5 und 6 nicht dargestellten Permanentmagneten innerhalb einer hermetischen dichten Hülle mechanisch gekoppelt. Mittels eines weiteren Magneten außerhalb der hermetisch dichten Hülle können der Permanentmagnet innerhalb der hermetisch dichten Hülle und mittels diesem die verschiebbare Korrekturlinse 57 bewegt werden, um die Korrekturwirkung der Korrekturlinsen 56, 57 zu verändern. In den Figuren 5 und 6 ebenfalls nicht dargestellte mechanische Anschläge können vorgesehen sein, um zwei vorbestimmte Positionen der verschiebbaren Korrekturlinse 57 und entsprechend zwei vorbestimmte Korrekturwerte zu definieren, beispielsweise einerseits für Luft oder andere Gas und andererseits für Wasser oder Flüssigkeiten mit ähnlichem Brechungsindex.

### Bezugszeichen

- 10: Endoskop
- 12: distales Ende des Endoskops 10
- 14: proximales Ende des Endoskops 10
- 16: Schaft des Endoskops 10
- 18: Blickrichtung des Endoskops 10
- 20: Fensterbauteil am distalen Ende 14 des Endoskops 10
- 28: Zylinderachse des Fensterbauteils 40
- 32: Schwenkprisma am distalen Ende 14 des Endoskops 10
- 34: Objektiv
- 36: Stablinse im Schaft 16 des Endoskops 10
- 37: Stablinse im Schaft 16 des Endoskops 10
- 40: Okular am proximalen Ende 12 des Endoskops 10^
- 41: erste (distale) Linse des Okulars 40
- 42: zweite (proximale) Linse des Okulars 40
- 48: optische Achse des Objektivs 34, der Stablinsen 36, 37 und des Okulars 40
- 52: Korrekturlinse am Objektiv 34
- 53: Korrekturlinse im Stablinsensystem 30
- 54: Korrekturlinse im oder am Okular 40
- 56: erste Korrekturlinse
- 57: zweite Korrekturlinse

## Patentansprüche

1. Endoskop (10), mit:
einem Fensterbauteil (20) aus einem transparenten Material am distalen Ende (12) des Endoskops (10), wobei das Fensterbauteil (20) nicht rotationssymmetrisch zur Blickrichtung (18) ist;
einer optischen Korrektureinrichtung (52, 53, 54, 56, 57) zur Korrektur der Aberration des Fensterbauteils (20),
wobei die Korrektureinrichtung eine Zylinderlinse (52, 53, 54, 56, 57) umfasst.

2. Endoskop (10) nach dem vorangehenden Anspruch, bei dem die Blickrichtung (18) des Endoskops einstellbar ist.

3. Endoskop (10) nach einem der vorangehenden Ansprüche, bei dem das Fensterbauteil (20) die Gestalt eines Ausschnitts eines Kreiszylindermantels aufweist.

4. Endoskop (10) nach einem der vorangehenden Ansprüche, bei dem die Korrektureinrichtung (52) nahe dem Fensterbauteil (20) angeordnet ist.

5. Endoskop (10) nach einem der vorangehenden Ansprüche, ferner mit:
einer schwenkbaren optischen Einrichtung (32) zum Einstellen der Blickrichtung (18) des Endoskops (10),
wobei die Korrektureinrichtung (52) proximal der schwenkbaren optischen Einrichtung (32) angeordnet ist.

6. Endoskop (10) nach einem der Ansprüche 1 bis3, ferner mit:
einem Objektiv (34) am distalen Ende (12) des Endoskops (10),
wobei die Korrektureinrichtung (52) in das Objektiv (34) integriert oder unmittelbar distal oder proximal des Objektivs (34) angeordnet ist.

7. Endoskop (10) nach einem der Ansprüche 1 bis3, ferner mit:
einem Relaislinsensystems (36, 37) zur Übertragung eines Bilds vom distalen Ende (12) zum proximalen Ende (14) des Endoskops (10),
wobei die Korrektureinrichtung (53) in das Relaislinsensystem (36, 37) integriert oder zwischen zwei Teilen (36, 37) des Relaislinsensystems (36, 37) angeordnet ist.

8. Endoskop (10) nach einem der Ansprüche 1 bis3, bei dem die Korrektureinrichtung (54; 56, 57) an oder nahe dem proximalen Ende (14) des Endoskops (10) angeordnet ist.

9. Endoskop (10) nach einem der Ansprüche 1 bis3, ferner mit:
eine Okular (40) am proximalen Ende (14) des Endoskops (10),
wobei die Korrektureinrichtung (54) in das Okular (40) integriert oder unmittelbar distal oder unmittelbar proximal des Okulars (40) angeordnet ist.

10. Endoskop (10) nach einem der vorangehenden Ansprüche, bei dem die Korrektureinrichtung (56, 57) eine einstellbare Korrekturwirkung aufweist, um eine Anpassbarkeit der Korrekturwirkung an den Brechungsindex eines Mediums, in dem das distale Ende (12) des Endoskops (10) angeordnet ist, zu ermöglichen.

11. Endoskop (10) nach dem vorangehenden Anspruch, bei dem die Korrekturwirkung der Korrektureinrichtung (56, 57) umschaltbar ist zwischen einer ersten vorbestimmten Korrekturwirkung für die Verwendung des distalen Endes (12) des Endoskops (10) in Luft oder einem anderen Gas und einer zweiten vorbestimmten Korrekturwirkung für die Verwendung des distalen Endes (12) des Endoskops (10) in Wasser oder einer Flüssigkeit mit ähnlichem Brechungsindex.

## Claims

1. Endoscope (10) comprising:
a window component (20) made of a transparent material at the distal end (12) of the endoscope (10), wherein the window component (20) is not rotationally symmetric in relation to the direction of view (18);
an optical correction apparatus (52, 53, 54, 56, 57) for correcting the aberration of the window component (20),
wherein the correction apparatus comprises a cylinder lens (52, 53, 54, 56, 57).

2. Endoscope (10) according to the preceding claim, wherein the direction of view (18) of the endoscope is adjustable.

3. Endoscope (10) according to one of the preceding claims, wherein the window component (20) has the form of a section of a circular cylinder barrel.

4. Endoscope (10) according to one of the preceding claims, wherein the correction apparatus (52) is disposed in the vicinity of the window component (20) .

5. Endoscope (10) according to one of the preceding claims, furthermore comprising:
a swivelable optical apparatus (32) for adjusting the direction of view (18) of the endoscope (10),
wherein the correction apparatus (52) is disposed proximally of the swivelable optical apparatus (32) .

6. Endoscope (10) according to one of Claims 1 to 3, furthermore comprising:
an objective (34) at the distal end (12) of the endoscope (10),
wherein the correction apparatus (52) is integrated into the objective (34) or disposed immediately distally or proximally of the objective (34).

7. Endoscope (10) according to one of Claims 1 to 3, furthermore comprising:
a relay lens system (36, 37) for transmitting an image from the distal end (12) to the proximal end (14) of the endoscope (10),
wherein the correction apparatus (53) is integrated into the relay lens system (36, 37) or disposed between two parts (36, 37) of the relay lens system (36, 37).

8. Endoscope (10) according to one of Claims 1 to 3, wherein the correction apparatus (54; 56, 57) is disposed on, or in the vicinity of, the proximal end (14) of the endoscope (10).

9. Endoscope (10) according to one of Claims 1 to 3, furthermore comprising:
an eyepiece (40) at the proximal end (14) of the endoscope (10),
wherein the correction apparatus (54) is integrated into the eyepiece (40) or disposed immediately distally or immediately proximally of the eyepiece (40) .

10. Endoscope (10) according to one of the preceding claims, wherein the correction apparatus (56, 57) has an adjustable corrective effect in order to enable an adaptability of the corrective effect to the refractive index of a medium, in which the distal end (12) of the endoscope (10) is disposed.

11. Endoscope (10) according to the preceding claim, wherein the corrective effect of the correction apparatus (56, 57) can be switched between a first predetermined corrective effect for the use of the distal end (12) of the endoscope (10) in air or any other gas and a second predetermined corrective effect for the use of the distal end (12) of the endoscope (10) in water or a liquid with a similar refractive index.

## Revendications

1. Endoscope (10), comprenant :
un composant formant fenêtre (20) constitué d'un matériau transparent à l'extrémité distale (12) de l'endoscope (10), dans lequel le composant formant fenêtre (20) n'est pas symétrique de rotation par rapport à la direction d'observation (18) ;
un dispositif de correction optique (52, 53, 54, 56, 57) destiné à corriger l'aberration du composant formant fenêtre (20), dans lequel le dispositif de correction comprend une lentille cylindrique (52, 53, 54, 56, 57).

2. Endoscope (10) selon la revendication précédente, dans lequel la direction d'observation (18) de l'endoscope est réglable.

3. Endoscope (10) selon la revendication précédente, dans lequel le composant formant fenêtre (20) présente la configuration d'une découpe ménagée dans une enveloppe cylindrique circulaire.

4. Endoscope (10) selon l'une des revendications précédentes, dans lequel le dispositif de correction (52) est disposé à proximité du composant formant fenêtre (20).

5. Endoscope (10) selon l'une des revendications précédentes, comportant en outre :
un dispositif optique pivotant (32) destiné à régler la direction d'observation (18) de l'endoscope (10),
dans lequel le dispositif de correction (52) est disposé à la position proximale du dispositif optique pivotant (32).

6. Endoscope (10) selon l'une des revendications 1 à 3, comportant en outre :
un objectif (34) à l'extrémité distale (12) de l'endoscope (10),
dans lequel le dispositif de correction (52) est intégré à l'objectif (34) ou est disposé directement à la position distale ou proximale de l'objectif (34).

7. Endoscope (10) selon l'une des revendications 1 à 3, comportant en outre :
un système de lentilles relais (36, 37) destiné à transmettre une image de l'extrémité distale (12) vers l'extrémité proximale (14) de l'endoscope (10),
dans lequel le dispositif de correction (53) est intégré au système de lentilles relais (36, 37) ou est disposé entre deux parties (36, 37) du système de lentilles relais (36, 37).

8. Endoscope (10) selon l'une des revendications 1 à 3, dans lequel le dispositif de correction (54 ; 56, 57) est disposé sur ou à proximité de l'extrémité proximale (14) de l'endoscope (10).

9. Endoscope (10) selon l'une des revendications 1 à 3, comportant en outre :
un oculaire (40) à l'extrémité proximale (14) de l'endoscope (10),
dans lequel le dispositif de correction (54) est intégré à l'oculaire (40) ou est disposé directement à la position distale ou directement à la position proximale de l'oculaire (40).

10. Endoscope (10) selon l'une des revendications précédentes, dans lequel le dispositif de correction (56, 57) présente un effet de correction réglable destiné à permettre une adaptabilité de l'effet de correction à l'indice de réfraction d'un milieu dans lequel est disposé l'extrémité distale (12) de l'endoscope (10).

11. Endoscope (10) selon la revendication précédentes, dans lequel l'effet de correction du dispositif de correction (56, 57) peut être amené à basculer entre un premier effet de correction prédéterminé permettant l'utilisation de l'extrémité distale (12) de l'endoscope (10) dans de l'air ou dans un autre gaz et un second effet de correction prédéterminé correspondant à l'utilisation de l'extrémité distale (12) de l'endoscope (10) dans de l'eau ou dans un autre liquide présentant un indice de réfraction semblable.
